# EUROPEAN PATENT SPECIFICATION

(11) **EP 3 406 191 B1**
(45) Date of publication and mention of the grant of the patent: **07.10.2020**
(21) Application number: 17741352.3
(22) Date of filing: 16.01.2017
(51) Int. Cl.: A61B 5/0484

(54) **MATERIAL EVALUATION METHOD AND MATERIAL EVALUATION DEVICE**
MATERIALBEURTEILUNGSVERFAHREN UND MATERIALBEURTEILUNGSVORRICHTUNG
MÉTHODE D'ÉVALUATION D'UN MATÉRIEL ET DISPOSITIF D'ÉVALUATION D'UN MATÉRIEL

(30) Priority: 18.01.2016 JP 2016007314
(43) Date of publication of application: 28.11.2018
(73) Proprietor: National Institute of Information and Communications Technology, Koganei-shi Tokyo 184-8795 (JP)
(72) Inventor: NISHIMOTO Shinji, Koganei-shi Tokyo 184-8795 (JP); NISHIDA Satoshi, Koganei-shi Tokyo 184-8795 (JP); KASHIOKA Hideki, Koganei-shi Tokyo 184-8795 (JP)
(74) Representative: EP&C
(86) International application number: PCT/JP2017/001253
(87) International publication number: WO 2017/126474

(56) References cited:
- WO-A1-2014/092045
- JP-A- 2008 102 594
- JP-A- 2015 207 038
- FRANCISCO PEREIRA ET AL: "Using Wikipedia to learn semantic feature representations of concrete concepts in neuroimaging experiments", ARTIFICIAL INTELLIGENCE., vol. 194, 1 January 2013 (2013-01-01), pages 240-252, XP55598103, NL ISSN: 0004-3702, DOI: 10.1016/j.artint.2012.06.005
- ALEXANDER G. HUTH ET AL: "A Continuous Semantic Space Describes the Representation of Thousands of Object and Action Categories across the Human Brain", NEURON, vol. 76, no. 6, 1 December 2012 (2012-12-01), pages 1210-1224, XP55597889, US ISSN: 0896-6273, DOI: 10.1016/j.neuron.2012.10.014
- LUEPOL PIPANMAEKAPORN ET AL: "Designing semantic feature spaces for brain-reading", ESANN 2015 PROCEEDINGS, 1 April 2015 (2015-04-01), pages 433-438, XP055598102, ISBN: 978-2-87587-014-8

## Description

### TECHNICAL FIELD

The present invention relates to a material evaluating method and a material evaluating apparatus.

### BACKGROUND ART

In recent years, technologies for estimating the semantic content of perception acquired by a test subject by measuring the brain activity of the test subject have come to be known (for example, Patent Document 1). In a technology disclosed in Patent Document 1, materials of video/audio content, various products, and the like are evaluated in the following process. In the conventional technology disclosed in Patent Document 1, first, a stimulus set configured of a moving image and the like is presented to a test subject, and brain activity caused by such a stimulus is measured. Next, in the conventional technology, for set data of a stimulus and brain activity, an intracerebral semantic space that is an intermediate representation of a correspondence relation is defined, and the correspondence relation between brain activity and the intracerebral semantic space is learned from the set data described above. Then, in the conventional technology, by using the learned correspondence relation, a position in the intracerebral semantic space is estimated from brain activity measured when a material of an evaluation target is presented to the test subject, and the material of the evaluation target is evaluated on the basis of the position in the intracerebral semantic space estimated from the brain activity.

### [Documents of the Prior Art]

### [Patent Document]

[Patent Document 1] Japanese Patent Application No. 2015-077694

FRANCISCO PEREIRA ET AL: "Using Wikipedia to learn semantic feature representations of concrete concepts in neuroimaging experiments", ARTIFICIAL INTELLIGENCE., vol. 194, 1 January 2013 (2013-01-01), pages 240-252, XP55598103, NL ISSN: 0004-3702, DOI: 10.1016/j.artint.2012.06.005, discloses using a corpus of a few thousand Wikipedia articles about concrete or visualizable concepts to produce a low-dimensional semantic feature representation of those concepts. The purpose of such a representation is to serve as a model of the mental context of a subject during functional magnetic resonance imaging (fMRI) experiments.

ALEXANDER G HUTH ET AL: "A Continuous Semantic Space Describes the Representation of Thousands of Object and Action Categories across the Human Brain", NEURON, vol. 76, no. 6, 1 December 2012 (2012-12-01), pages 1210-1224, XP55597889 US ISSN: 0896-6273, DOI: 10.1016/j.neuron.2012.10.014 discloses to represent object and action categories as locations in a continuous semantic space mapped across a cortical surface. To search for such a space, fMRl is used to measure human brain activity evoked by natural movies. Voxelwise models are used to examine the cortical representation of object and action categories.

LUEPOL PIPANMAEKAPORN ET AL: "Designing semantic feature spaces for brain-reading", ESANN 2015 PROCEEDINGS, 1 April 2015 (2015-04-01), pages 433-438, XP055598102, ISBN: 978-2-87587-014-8 discloses a brain reading task which consists in discovering a word a person is thinking of based on an fMRI image of the brain. Several semantic spaces are automatically designed from linguistic resources and combined in a principled way, achieving results comparable to the results of manually built semantic spaces.

### SUMMARY OF INVENTION

### [Problems to be Solved by the Invention]

However, in the conventional technology, whenever there is a request for evaluating a new material, it is necessary to present the new material to the test subject and measure brain activity, and thus it is difficult to make an evaluation quickly and easily.

The present invention is for solving the problem described above, and an object thereof is to provide a material evaluating method and a material evaluating apparatus capable of making an evaluation quickly and easily.

### [Means for Solving the Problems]

In order to solve the above-described problem, according to the present invention, there is provided a material evaluating method including: a brain activity measuring step of presenting a training material to a test subject and measuring brain activity by using a brain activity measuring unit; a semantic space building step of building an intracerebral semantic space representing an intracerebral semantic relation between the brain activity and a word appearing in a language description on the basis of a measurement result acquired in the brain activity measuring step and the language description acquired by performing natural language processing for content of the training material by using a semantic space building unit; a first estimation step of estimating a first position corresponding to a content of new material in the intracerebral semantic space from a language description acquired by performing natural language processing for the content of the new material by using a material estimating unit; a second estimation step of estimating a second position corresponding to an object word in the intracerebral semantic space from the object word representing an object concept of the new material by using an object estimating unit; and an evaluation step of evaluating the new material on the basis of the first position estimated in the first estimation step and the second position estimated in the second estimation step by using an evaluation processing unit.

In addition, according to a preferred embodiment of the present invention, there is provided a material evaluating method in which, in the evaluation step of the material evaluating method described above, the evaluation processing unit evaluates whether or not the new material is close to the object concept on the basis of a distance between the first position and the second position.

Furthermore, according to a preferred embodiment of the present invention, there is provided a material evaluating method in which, in the evaluation step of the material evaluating method described above, the evaluation processing unit evaluates whether or not the new material is close to the object concept by using an inner product value of a vector representing the first position and a vector representing the second position as an index.

In addition, according to a preferred embodiment of the present invention, there is provided a material evaluating method in which, in the first estimation step of the material evaluating method described above, the material estimating unit estimates a position in the intracerebral semantic space that corresponds to each of words included in the language description as the first position.

Furthermore, according to a preferred embodiment of the present invention, there is provided a material evaluating method in which, in the first estimation step of the material evaluating method described above, the material estimating unit estimates a position in the intracerebral semantic space for each of words included in the language description and estimates the gravity center of the positions of the words as the first position.

In addition, according to the present invention, there is provided a material evaluating apparatus including: a semantic space storing unit that stores intracerebral semantic space information representing an intracerebral semantic space built on the basis of a measurement result of brain activity acquired by presenting a training material to a test subject using a brain activity measuring unit and a language description acquired by performing natural language processing for content of the training material and representing an intracerebral semantic relation between the brain activity and words appearing in the language description; a material estimating unit estimating a first position corresponding to content of a new material in the intracerebral semantic space from a language description acquired by performing natural language processing for the content of the new material on the basis of the intracerebral semantic space information stored by the semantic space storing unit; an object estimating unit estimating a second position corresponding to an object word in the intracerebral semantic space from the object word representing an object concept of the new material on the basis of the intracerebral semantic space information stored by the semantic space storing unit; and an evaluation processing unit evaluating the new material on the basis of the first position estimated by the material estimating unit and the second position estimated by the object estimating unit.

### [Advantageous Effects of the Invention]

According to the present invention, brain activity does not need to be newly measured for an evaluation of a new material, and therefore an evaluation can be made quickly and easily.

### BRIEF DESCRIPTION OF DRAWINGS

Fig. 1 is a functional block diagram illustrating an example of a material evaluating system according to this embodiment.
Fig. 2 is a diagram illustrating an example of building an intracerebral semantic space according to this embodiment.
Fig. 3 is a diagram illustrating an example of a material evaluating process according to this embodiment.
Fig. 4 is a flowchart illustrating an example of the operation of a material evaluating system according to this embodiment.
Fig. 5 is a diagram illustrating an example of a result of an evaluation made by a material evaluating system according to this embodiment.

### EMBODIMENTS FOR CARRYING OUT THE INVENTION

Hereinafter, a material evaluating system and a material evaluating method according to one embodiment of the present invention will be described with reference to the drawings.

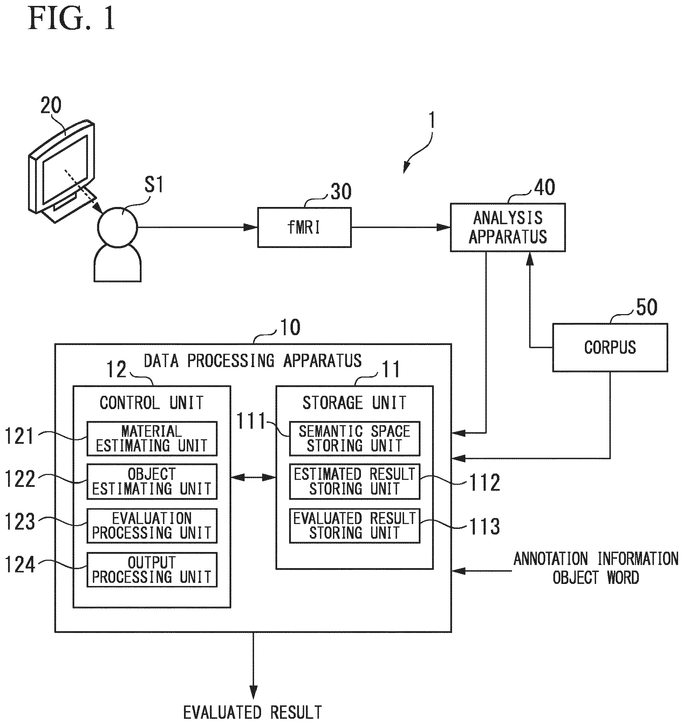

Fig. 1 is a functional block diagram illustrating an example of a material evaluating system 1 according to this embodiment.

As illustrated in Fig. 1, the material evaluating system 1 includes a data processing apparatus 10, an image reproducing terminal 20, a functional magnetic resonance imaging (fMRI) 30, and an analysis apparatus 40.

The material evaluating system 1 according to this embodiment performs estimation of perceived content, a purchase prediction, and the like (hereinafter referred to as evaluation) relating to a material that is video/audio content, any kind of product, or the like of a CM moving image (commercial moving image; commercial film (CF)).

The image reproducing terminal 20, for example, is a terminal device including a liquid crystal display or the like and, for example, displays a moving image for training (training moving image) or the like and allows a test subject S1 to view the displayed moving image.

Here, the training moving image (an example of a training material) is a moving image including a wide variety of images.

The fMRI 30 (an example of a brain activity measuring unit) measures brain activity of the test subject S1 who has viewed an image (for example, a training moving image or the like) displayed by the image reproducing terminal 20. In other words, the fMRI 30 measures the brain activity of the test subject S1 for a stimulus to the test subject S1 that is given by presenting the training moving image to the test subject S1. The fMRI 30 outputs an fMRI signal (brain activity signal) that visualizes a hemodynamic reaction relating to brain activity of the test subject S1. The fMRI 30 measures the brain activity of the test subject S1 at a predetermined time interval (for example, a two-second interval) and outputs a measurement result to the analysis apparatus 40 as an fMRI signal.

The analysis apparatus 40 (an example of a semantic space building unit) builds an intracerebral semantic space representing an intracerebral semantic relation between brain activity and a word appearing in a language description on the basis of a measurement result acquired by the fMRI 30 and the language description (annotation) acquired by performing natural language processing for the content of the training moving image.

The analysis apparatus 40, for example, defines an intracerebral semantic space that is an intermediate representation of a correspondence relation for set data of a stimulus according to the training moving image and brain activity by using a statistical learning model. The analysis apparatus 40 outputs intracerebral semantic space information representing the built intracerebral semantic space to the data processing apparatus 10 to store the intracerebral semantic space information in a semantic space storing unit 111 of the data processing apparatus 10.

The data processing apparatus 10 (an example of a material evaluating apparatus) is a computer apparatus that evaluates a new material of an evaluation target on the basis of the intracerebral semantic space built by the analysis apparatus 40 without newly measuring brain activity using the fMRI 30. The data processing apparatus 10 projects semantic content of a new material into the intracerebral semantic space built by the analysis apparatus 40 and projects an object word representing an object concept into the intracerebral semantic space. Then, the data processing apparatus 10 evaluates the new material on the basis of a position (first position) in the intracerebral semantic space corresponding to the new material and a position (second position) in the intracerebral semantic space corresponding to the object word. The data processing apparatus 10 includes a storage unit 11 and a control unit 12.

The storage unit 11 stores various kinds of information used for various processes performed by the data processing apparatus 10. The storage unit 11 includes a semantic space storing unit 111, an estimated result storing unit 112, and an evaluated result storing unit 113.

The semantic space storing unit 111 stores the intracerebral semantic space information representing the intracerebral semantic space built by the analysis apparatus 40. Here, the intracerebral semantic space information, for example, is a projection function projecting an annotation vector to be described later into the intracerebral semantic space.

The estimated result storing unit 112 stores estimated results acquired using a material estimating unit 121 and an object estimating unit 122 to be described later. The estimated result storing unit 112, for example, stores an estimated result representing a position in the intracerebral semantic space that corresponds to a new material and an estimated result representing a position in the intracerebral semantic space that corresponds to an object word.

The evaluated result storing unit 113 stores an evaluated result of a new material. The evaluated result storing unit 113, for example, stores information of an index of a distance in the intracerebral semantic space to be described later and the like.

The control unit 12, for example, is a processor including a central processing unit (CPU) or the like and integrally controls the data processing apparatus 10. The control unit 12 performs various processes performed by the data processing apparatus 10. The control unit 12 includes a material estimating unit 121, an object estimating unit 122, an evaluation processing unit 123, and an output processing unit 124.

The material estimating unit 121 estimates a position (first position) corresponding to the content of a new material in the intracerebral semantic space from a language description acquired by performing natural language processing for the content of the new material. In other words, the material estimating unit 121 estimates a first position corresponding to the content of a new material in the intracerebral semantic space from a language description acquired by performing natural language processing for the content of the new material on the basis of the intracerebral semantic space information stored by the semantic space storing unit 111. Here, the language description, for example, is an annotation vector to be described later. The material estimating unit 121 projects an annotation vector corresponding to the content of the new material into the intracerebral semantic space by using the intracerebral semantic space information.

Here, the process of generating an annotation vector from the content of the new material by using the material estimating unit 121 will be described. When the new material is, for example, an image, text (annotation information) of a language description representing the impression of the image is generated in advance, and the material estimating unit 121 acquires the annotation information from the outside. Here, the annotation information, for example, is text of a description, a feeling, or the like of a scene overview (an overview of an image) of 50 characters to 150 characters. The material estimating unit 121 performs a morpheme analysis of acquired text (annotation information) of the language description, for example, by using MeCab or the like, thereby generating spaced word data. Next, the material estimating unit 121 performs natural language processing (for example, word2vec) for each word included in the spaced word data by using a corpus 50, thereby generating an annotation vector that is a matrix such as Skip-gram or the like for each word.

Here, the corpus 50 is, for example, a database of a large amount of text data such as Wikipedia (registered trademark), newspaper articles, or the like. The material estimating unit 121 performs natural language processing for such a large amount of text data as the corpus 50, thereby generating annotation vectors. Here, an annotation vector is a result of calculation of a word registered in the corpus 50 having a shortest distance (meaning) according to the relation for a word appearing in the language description (annotation) representing the impression of an image.

The material estimating unit 121, for example, translates (projects) an annotation vector to a position in the intracerebral semantic space on the basis of the intracerebral semantic space information described above for each generated word. The material estimating unit 121 may set each position corresponding to each projected word as the first position described above or may set the gravity center (mean) of positions corresponding to projected words as the first position described above.

In addition, the material estimating unit 121 stores an estimated result representing the position (first position) (for example, a vector (V1) representing the position) in the intracerebral semantic space that corresponds to the content of the new material in the estimated result storing unit 112.

The object estimating unit 122 estimates a position (second position) corresponding to an object word in the intracerebral semantic space from the object word representing the object concept of the new material. In other words, the object estimating unit 122 estimates a second position corresponding to an object word in the intracerebral semantic space from the object word on the basis of the intracerebral semantic space information stored by the semantic space storing unit 111. Here, an object word, for example, is a word representing the concept of an object of the new material of the evaluation target such as "lovely," "fresh," or the like.

The object estimating unit 122 acquires an object word from the outside and performs natural language processing (for example, word2vec) for the object word by using the corpus 50, thereby generating an annotation vector corresponding to the object word that is a matrix of Skip-gram or the like. The object estimating unit 122 translates (projects) the annotation vector corresponding to the object word to a position in the intracerebral semantic space on the basis of the intracerebral semantic space information described above.

In addition, the object estimating unit 122 stores an estimated result representing a position (second position) (for example, a vector (V2) representing the position) in the intracerebral semantic space that corresponds to the object word in the estimated result storing unit 112.

The evaluation processing unit 123 evaluates a new material on the basis of the first position estimated by the object estimating unit 122 and the second position estimated by the object estimating unit 122. The evaluation processing unit 123, for example, evaluates whether or not the new material is close to the object concept on the basis of a distance between the first position and the second position in the intracerebral semantic space. In other words, the evaluation processing unit 123 can regard the new material as closer to the object concept when the distance in the intracerebral semantic space is shorter and can determine that the production intention is reflected.

On the other hand, the evaluation processing unit 123 can regard the new material as farther from the object concept when the distance in the intracerebral semantic space is longer and can determine that the production intention is not reflected.

In addition, the evaluation processing unit 123, for example, calculates a Euclidean distance, Mahalanobis distance, entropy, likelihood, or the like as an index of the distance in the intracerebral semantic space. The evaluation processing unit 123 may evaluate whether or not a new material is close to the object concept by using an inner product value (V1·V2) of a vector (V1) representing a first position corresponding to the content of the new material and a vector (V2) representing a second position corresponding to the object word as an index.

Furthermore, the evaluation processing unit 123 stores information of the index of the calculated distance in the intracerebral semantic space or the like in the evaluated result storing unit 113.

The output processing unit 124 outputs an evaluated result acquired by the evaluation processing unit 123 to the outside. The output processing unit 124, for example, forms the information of an index or the like of the distance stored by the evaluated result storing unit 113 as a graph and outputs the graph or the like.

Next, the operation of the material evaluating system 1 according to this embodiment will be described with reference to the drawings.

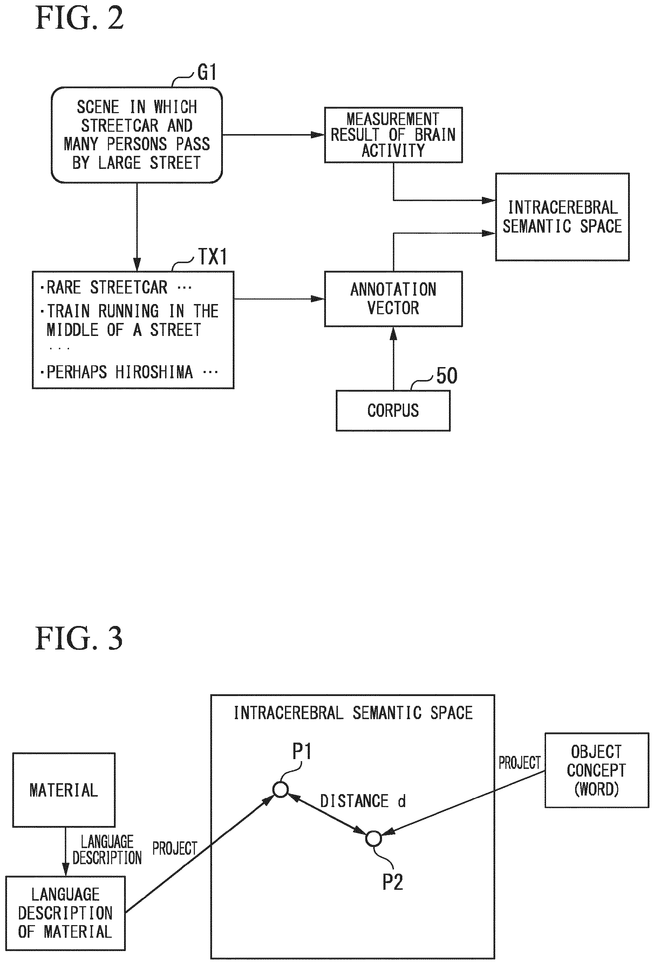

First, the process of building an intracerebral semantic space according to this embodiment will be described with reference to Fig. 2.

Fig. 2 is a diagram illustrating an example of building an intracerebral semantic space according to this embodiment.

As illustrated in Fig. 2, first, a training moving image is displayed on the image reproducing terminal 20, and the brain activity acquired when the test subject S1 views the training moving image is measured by the fMRI 30. Accordingly, a measurement result of the brain activity is acquired. The analysis apparatus 40 acquires the measurement result of the brain activity acquired by the fMRI 30.

In addition, annotation information (for example, annotation information TX1) is generated from a scene image (for example, an image G1) included in the training moving image. For example, when the scene image G1 is a "scene in which a street car and many persons pass by a large street," the annotation information TX1 is text describing an overview of the scene such as "a rare street car ···," "a train running in the middle of a street ···," "perhaps Hiroshima ···," or the like. This annotation information is similar to the text of the language description described in the material estimating unit 121 described above.

In addition, the analysis apparatus 40 performs natural language processing on the basis of the corpus 50 and generates an annotation vector from the annotation information. The analysis apparatus 40, similar to the description of the object estimating unit 122, for example, performs a morpheme analysis of the annotation information by using MeCab or the like, thereby generating spaced word data. Next, the analysis apparatus 40 performs natural language processing (for example, word2vec) for each word included in the spaced word data by using the corpus 50, thereby generating an annotation vector that is a matrix such as the Skip-gram for each word. The annotation vector used for building the intracerebral semantic space here is, for example, an annotation vector for each word included in the annotation information.

Next, the analysis apparatus 40 performs a statistical learning process by using a set of the measurement result of the brain activity and the annotation vector, thereby building an intracerebral semantic space. The analysis apparatus 40, for example, performs a statistical learning process such as a regression model or the like, thereby building an intracerebral semantic space representing an intracerebral semantic relation between brain activity and a word appearing in the language description. The analysis apparatus 40 outputs intracerebral semantic space information representing the built intracerebral semantic space to the data processing apparatus 10 to store the intracerebral semantic space information in the semantic space storing unit 111.

Next, a material evaluating process according to this embodiment will be described with reference to Fig. 3.

Fig. 3 is a diagram illustrating an example of the material evaluating process according to this embodiment.

As illustrated in Fig. 3, first, the material estimating unit 121 of the data processing apparatus 10 projects a language description (annotation information) of a material having the content of the material (for example, a moving image of the evaluation target or the like) as the language description into the intracerebral semantic space. In other words, the material estimating unit 121 generates spaced word data by performing a morpheme analysis of the annotation information and performs natural language processing (for example, word2vec) for each word included in the spaced word data by using the corpus 50, thereby generating an annotation vector that is a matrix such as Skip-gram for each word.

Next, the material estimating unit 121 projects the generated annotation vectors to a position P1 corresponding to the content of the new material in the intracerebral semantic space on the basis of the intracerebral semantic space information stored by the semantic space storing unit 111. More specifically, the material estimating unit 121 projects the annotation vector for each word into the intracerebral semantic space on the basis of the intracerebral semantic space information such that the gravity center of projection positions is a position P1 corresponding to the content of the new material.

In addition, the object estimating unit 122 of the data processing apparatus 10 projects an object word that is the object concept of the material into the intracerebral semantic space. In other words, the object estimating unit 122 performs natural language processing (for example, word2vec) for an object word by using the corpus 50, thereby generating an object concept vector corresponding to the object word that is a matrix such as Skip-gram. The object estimating unit 122 projects an object concept vector corresponding to the object word to a position P2 corresponding to the object word in the intracerebral semantic space on the basis of the intracerebral semantic space information stored by the semantic space storing unit 111.

Next, the evaluation processing unit 123 of the data processing apparatus 10 evaluates the material on the basis of a distance d between the position P1 and the position P2 in the intracerebral semantic space described above. For example, as the distance d is shorter, it represents that the test subject S1 perceives the content of the new material of the evaluation target as content closer to the object word in his or her brain, and the evaluation processing unit 123 determines that the new material reflects the production intention more when the distance d is shorter. On the other hand, for example, as the distance d is longer, it represents that the test subject S1 perceives the content of the new material of the evaluation target as content farther from the object word in his or her brain, and the evaluation processing unit 123 determines that the new material of the evaluation target reflects the production intention less when the distance d is longer.

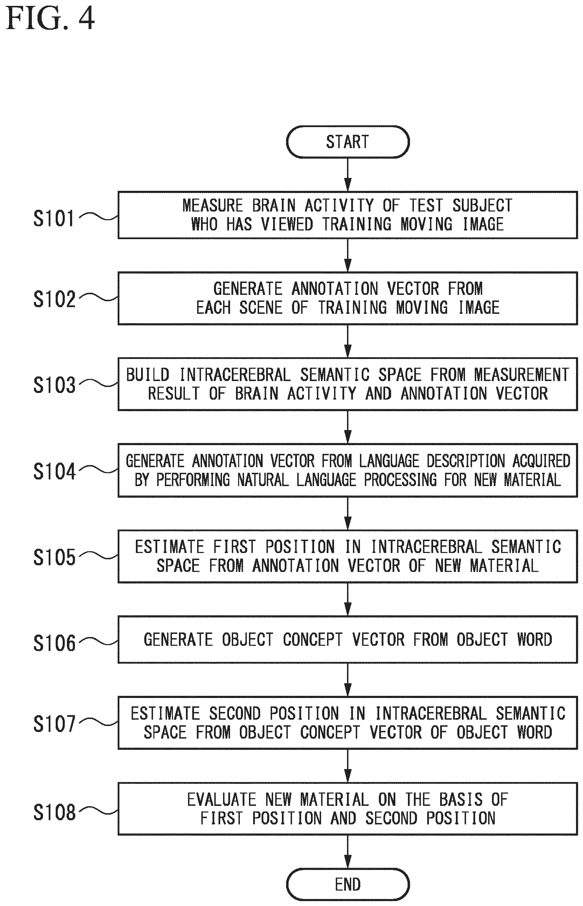

Next, the whole operation of the material evaluating system 1 according to this embodiment will be described with reference to Fig. 4.

Fig. 4 is a flowchart illustrating an example of the operation of the material evaluating system 1 according to this embodiment.

As illustrated in Fig. 4, the material evaluating system 1 first measures brain activity of a test subject who has viewed a training moving image (Step S101). In other words, the image reproducing terminal 20 of the material evaluating system 1 displays the training moving image, and the fMRI 30 measures the brain activity of the test subject S1 who has viewed the training moving image. The fMRI 30 outputs a measurement result acquired through the measurement to the analysis apparatus 40.

Next, the analysis apparatus 40 generates an annotation vector from each scene of the training moving image (Step S102). The analysis apparatus 40, as illustrated in Fig. 2, generates an annotation vector from each scene of the training moving image.

Next, the analysis apparatus 40 builds an intracerebral semantic space from the measurement result of the brain activity and the annotation vector (Step S103). The analysis apparatus 40 performs a statistical learning process for a set of the measurement result of the brain activity and the annotation vector and builds an intracerebral semantic space corresponding to the test subject S1 in which the brain activity and the annotation vector are associated with each other (see Fig. 2). The analysis apparatus 40 outputs intracerebral semantic space information representing the built intracerebral semantic space to the data processing apparatus 10 and stores the intracerebral semantic space information in the semantic space storing unit 111 of the data processing apparatus 10.

Next, the data processing apparatus 10 generates an annotation vector from the language description acquired by performing natural language processing for the new material that is the evaluation target (Step S104). The material estimating unit 121 of the data processing apparatus 10, for example, generates an annotation vector from each scene of a certain new material of a CM moving image.

Next, the material estimating unit 121 estimates a first position in the intracerebral semantic space from the annotation vector of the new material (Step S105). The material estimating unit 121, for example, as illustrated in Fig. 3, projects the annotation vector of the new material to a position P1 corresponding to the new material in the intracerebral semantic space. In addition, the material estimating unit 121, for example, projects the annotation vector corresponding to each scene into the intracerebral semantic space and estimates a plurality of positions P1.

Next, the data processing apparatus 10 generates an object concept vector from the object word (Step S106). The object estimating unit 122 of the data processing apparatus 10 generates an object concept vector from the object word (for example, "lovely" or the like) representing the object concept of the new material.

Next, the object estimating unit 122 estimates a second position in the intracerebral semantic space from the object concept vector of the object word (Step S107). The object estimating unit 122, for example, as illustrated in Fig. 3, projects the object concept vector of the object word to a position P2 corresponding to the object word in the intracerebral semantic space.

Next, the evaluation processing unit 123 of the data processing apparatus 10 evaluates the new material on the basis of the first position (the position PI) and the second position (the position P2) in the intracerebral semantic space (Step S108). The evaluation processing unit 123, for example, calculates an inner product value of the vector (V1) representing the position P1 corresponding to each scene and the vector (V2) representing the position P2 corresponding to the object word as an index of the evaluation.

In the flowchart of the material evaluation described above, the process of Step S101 corresponds to the process of a brain activity measuring step, and the process of Steps S102 and S103 corresponds to the process of a semantic space building step. In addition, the process of Steps S104 and S105 corresponds to the process of a first estimation step, the process of Steps S106 and S107 corresponds to the process of a second estimation step, and the process of Step S108 corresponds to an evaluation step. Here, although the process of Steps S101 to S103 includes the measurement of brain activity, the process of Steps S104 to S108 required for the evaluation of the new material does not include new measurement of brain activity. In this way, the material evaluating method according to this embodiment provides a quick and easy evaluation means not requiring new brain activity measurement for an evaluation request for a new material.

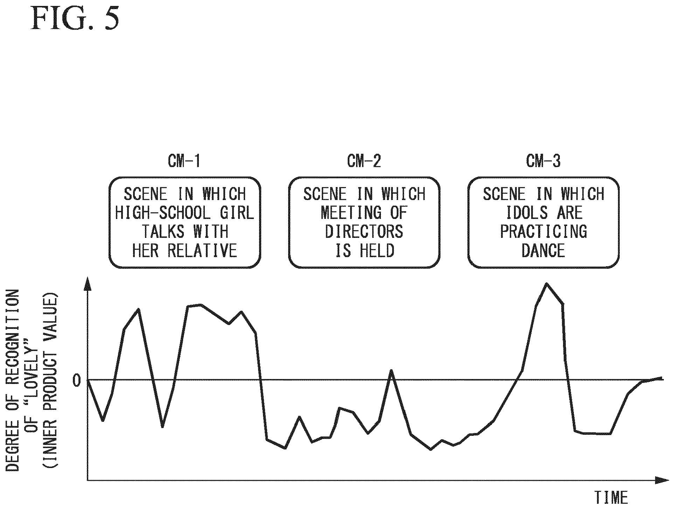

Next, an example of a result of an evaluation made by the material evaluating system 1 will be described with reference to Fig. 5.

Fig. 5 is a diagram illustrating an example of a result of an evaluation made by the material evaluating system 1 according to this embodiment.

The example illustrated in Fig. 5 is an example in which the data processing apparatus 10 according to this embodiment makes a quantitative evaluation of a specific CM moving image. This is for the purpose of provision of a quantitative index, for example, an index representing which one of two videos A and B has a stronger specific impression for a viewer or the like. For example, by configuring three 30-second CM moving images as new materials and checking the degree of recognition (here, an inner product value) of an object word (in this case, "lovely") representing an object concept in each scene of the CM moving image, the degree of perception of the object word is estimated.

In a graph illustrated in Fig. 5, the vertical axis represents the degree of recognition (inner product value) of "lovely," and the horizontal axis represents time.

For example, a CM moving image CM-1 is a "scene in which a high-school girl talks with her relative," a CM moving image CM-2 is a "scene in which a meeting of directors is held," and a CM moving image CM-3 is a "scene in which idols are practicing a dance."

In the example illustrated in Fig. 5, it is illustrated that the CM moving image CM-1 is a material that has a largest inner product value and is closest to the object word ("lovely").

In addition, in the example illustrated in Fig. 5, while the evaluation processing unit 123 makes an evaluation using an intracerebral semantic space corresponding to one test subject S1, a new material may be evaluated using intracerebral semantic spaces built using a plurality of test subjects S1. In such a case, the semantic space storing unit 111 associates identification information of each test subject S1 with intracerebral semantic space information.

In addition, when an evaluation target in accordance with a new material is known, such as in the case of a CM for women, a CM for children, or the like, the evaluation processing unit 123 may be configured to make an evaluation by changing the intracerebral semantic space information corresponding a test subject S1 in accordance with the type of new material of the evaluation target.

As described above, the material evaluating method according to this embodiment includes the brain activity measuring step, the semantic space building step, the first estimation step, the second estimation step, and the evaluation step. In the brain activity measuring step, the fMRI 30 (brain activity measuring unit) presents training material (for example, a training moving image) to a test subject S1 and measures brain activity. In the semantic space building step, the analysis apparatus 40 (semantic space building unit) builds an intracerebral semantic space representing an intracerebral semantic relation between brain activity and a word appearing in the language description on the basis of a measurement result acquired in the brain activity measuring step and a language description (annotation) acquired by performing natural language processing for the content of the training material. In the first estimation step, the material estimating unit 121 estimates a first position corresponding to the content of new material in the intracerebral semantic space from the language description acquired by performing natural language processing for the content of the new material. In the second estimation step, the object estimating unit 122 estimates a second position corresponding to an object word in the intracerebral semantic space from the object word representing the object concept of the new material. Then, in the evaluation step, the evaluation processing unit 123 evaluates new material on the basis of the first position estimated in the first estimation step and the second position estimated in the second estimation step.

In this way, the material evaluating method according to this embodiment can quantitatively evaluate whether or not a new material to be newly evaluated causes an intracerebral expression close to the object concept without performing newly measuring brain activity. In addition, according to the material evaluating method of this embodiment, when a new material is evaluated, individual brain activity does not need to be measured, and accordingly, the cycle of material generation and an evaluation can be reduced much. Therefore, according to the material evaluating method of this embodiment, an evaluation can be made quickly and easily. In addition, according to the material evaluating method of this embodiment, human costs, time costs, and monetary costs can be reduced.

Furthermore, in this embodiment, in the evaluation step, the evaluation processing unit 123 evaluates whether or not a new material is close to an object concept on the basis of a distance (d) between the first position (position PI) and the second position (position P2) on the intracerebral semantic space.

In this way, the material evaluating method according to this embodiment can make an evaluation (for example, estimation of perceived content, a purchase prediction, or the like) of a new material objectively and quantitatively.

In addition, in this embodiment, in the evaluation step, the evaluation processing unit 123 may evaluate whether or not a new material is close to an object concept by using an inner product value of a vector representing the first position and a vector representing the second position as an index.

In such a case, according to the material evaluating method of this embodiment, as illustrated in Fig. 5, a quantitative evaluation of a new material can be appropriately made by using a simple technique of the calculation of an inner product value.

In addition, in this embodiment, in the first estimation step, the material estimating unit 121 estimates a position in the intracerebral semantic space for each word included in the language description and estimates the gravity center of the positions of the words as the first position.

Thus, according to the material evaluating method of this embodiment, the first position corresponding to the new material in the intracerebral semantic space can be appropriately estimated by using a simple technique of calculation of the gravity center.

In addition, in this embodiment, in the first estimation step, the material estimating unit 121 may estimate a position in the intracerebral semantic space that corresponds to each word included in the language description as the first position.

In such a case, according to the material evaluating method of this embodiment, a distance up to the object word can be evaluated in units of words included in the language description representing the content of the new material.

In addition, the data processing apparatus 10 (material evaluating apparatus) and the material evaluating system 1 according to this embodiment includes the semantic space storing unit 111, the material estimating unit 121, the object estimating unit 122, and the evaluation processing unit 123. The semantic space storing unit 111 stores intracerebral semantic space information that represents an intracerebral semantic space built on the basis of measurement results of brain activity by presenting a training material to the test subject S1 by using the fMRI 30 and the language description acquired by performing natural language processing for the content of the training material and representing an intracerebral semantic relation between brain activity and a word appearing in the language description. The material estimating unit 121 estimates a first position corresponding to the content of a new material in the intracerebral semantic space from the language description acquired by performing natural language processing for the content of the new material on the basis of the intracerebral semantic space information stored by the semantic space storing unit 111. The object estimating unit 122 estimates a second position corresponding to an object words in the intracerebral semantic space from the object words representing the object concept of the new material on the basis of the intracerebral semantic space information stored by the semantic space storing unit 111. The evaluation processing unit 123 evaluates a new material on the basis of the first position estimated by the material estimating unit 121 and the second position estimated by the object estimating unit 122.

In this way, according to the data processing apparatus 10 and the material evaluating system 1 of this embodiment, similar to the material evaluating method described above, a new material can be quantitatively evaluated without measuring new brain activity and can be evaluated quickly and easily.

The present invention is not limited to the embodiment described above, and a change can be made in a range not departing from the concept of the present invention.

For example, in the embodiment described above, while an example in which the material evaluating system 1 includes the image reproducing terminal 20, the fMRI 30, and the analysis apparatus 40 has been described, in a case where the intracerebral semantic space information is stored in the semantic space storing unit 111 in advance, the material evaluating system may be configured only by the data processing apparatus 10. In addition, the data processing apparatus 10 may include the function of the analysis apparatus 40.

In addition, in the embodiment described above, while an example in which the output processing unit 124 outputs an evaluated result to the outside has been described, the configuration is not limited thereto, and the data processing apparatus 10 may include a display unit, and the evaluated result may be output to the display unit. Furthermore, all or part of the storage unit 11 may be provided outside the data processing apparatus 10.

In addition, in the embodiment described above, while an example in which each scene of the new material (CM moving image) is evaluated has been described, each cut of a storyboard or the like may be evaluated, and the whole content of the new material (CM moving image) may be evaluated.

Furthermore, in the embodiment described above, while an example in which the annotation vector used for building an intracerebral semantic space is an annotation vector of each word included in the annotation information has been described, an annotation vector using the gravity center of the annotation vector or each word for building an intracerebral semantic space may be used.

In addition, each configuration included in the data processing apparatus 10 and the analysis apparatus 40 described above includes an internal computer system. Then, by recording a program used for realizing the function of each configuration included in the data processing apparatus 10 and the analysis apparatus 40 described above on a computer-readable recording medium and causing the computer system to read and execute the program recorded on this recording medium, the process of each configuration included in the data processing apparatus 10 and the analysis apparatus 40 described above may be performed. Here, "the computer system is caused to read and execute the program recorded on the recording medium" includes a case in which the computer system is causes to install the program in the computer system. The "computer system" described here includes an OS and hardware such as peripherals.

In addition, the "computer system" may include a plurality of computer apparatuses connected through a network including the Internet, a WAN, a LAN or a communication line such as a dedicated line. Furthermore, the "computer-readable recording medium" represents a portable medium such as a flexible disc, a magneto-optical disk, a ROM, or a CD-ROM or a storage device such as a hard disk built in the computer system. In this way, the recording medium in which the program is stored may be a non-transient recording medium such as a CD-ROM.

In addition, the recording medium includes a recording medium installed inside or outside that is accessible from a distribution server for distributing the program. Furthermore, a configuration in which the program is divided into a plurality of parts, and the parts are downloaded at different timings and then are combined in each configuration included in the data processing apparatus 10 and the analysis apparatus 40 may be employed, and distribution servers distributing the divided programs may be different from each other. In addition, the "computer-readable recording medium" includes a medium storing the program for a predetermined time such as an internal volatile memory (RAM) of a computer system serving as a server or a client in a case in which the program is transmitted through a network. Furthermore, the program described above may be a program used for realizing part of the function described above. In addition, the program may be a program to be combined with a program that has already been recorded in the computer system for realizing the function described above, a so-called a differential file (differential program).

Furthermore, at least part of the function described above may be realized by an integrated circuit of a large scale integration (LSI) or the like. Each function described above may be individually configured as a processor, or at least part of the functions may be integrated and configured as a processor. In addition, a technique used for configuring the integrated circuit is not limited to the LSI, and each function may be realized by a dedicated circuit or a general-purpose processor. Furthermore, in a case in which a technology of configuring an integrated circuit replacing the LSI emerges in accordance with the progress of semiconductor technologies, an integrated circuit using such a technology may be used.

### [Reference Symbols]

- 1: Material evaluating system
- 10: Data processing apparatus
- 11: Storage unit
- 12: Control unit
- 20: Image reproducing terminal
- 30: fMRI
- 40: Analysis apparatus
- 50: Corpus
- 111: Semantic space storing unit
- 112: Estimated result storing unit
- 113: Evaluated result storing unit
- 121: Material estimating unit
- 122: Object estimating unit
- 123: Evaluation processing unit
- 124: Output Processing unit
- S1: Test subject

## Claims

1. A material evaluating method comprising:
a brain activity measuring step (S101) of presenting a training material to a test subject and measuring brain activity by using a brain activity measuring unit;
a semantic space building step (S103) of building an intracerebral semantic space representing an intracerebral semantic relation between the brain activity and a word appearing in a language description on the basis of a measurement result acquired in the brain activity measuring step and the language description acquired by performing natural language processing for content of the training material by using a semantic space building unit;
a first estimation step (S105) of estimating a first position corresponding to content of a new material in the intracerebral semantic space from a language description acquired by performing natural language processing for the content of the new material by using a material estimating unit; **characterized in that** the method further comprises:
a second estimation step (S107) of estimating a second position corresponding to an object word in the intracerebral semantic space from the object word representing an object concept of the new material by using an object estimating unit; and
an evaluation step (S108) of evaluating the new material on the basis of the first position estimated in the first estimation step and the second position estimated in the second estimation step by using an evaluation processing unit.

2. The material evaluating method according to claim 1,
wherein, in the evaluation step, the evaluation processing unit evaluates whether or not the new material is close to the object concept on the basis of a distance between the first position and the second position.

3. The material evaluating method according to claim 1,
wherein, in the evaluation step, the evaluation processing unit evaluates whether or not the new material is close to the object concept by using an inner product value of a vector representing the first position and a vector representing the second position as an index.

4. The material evaluating method according to any one of claims 1 to 3,
wherein, in the first estimation step, the material estimating unit estimates a position in the intracerebral semantic space that corresponds to each of words included in the language description as the first position.

5. The material evaluating method according to any one of claims 1 to 3,
wherein, in the first estimation step, the material estimating unit estimates a position in the intracerebral semantic space for each of words included in the language description and estimates the gravity center of the positions of the words as the first position.

6. A material evaluating apparatus comprising:
a semantic space storing unit (111) that stores intracerebral semantic space information representing an intracerebral semantic space built on the basis of a measurement result of brain activity acquired by presenting a training material to a test subject using a brain activity measuring unit and a language description acquired by performing natural language processing for content of the training material and representing an intracerebral semantic relation between the brain activity and words appearing in the language description;
a material estimating unit (121) for estimating a first position corresponding to content of a new material in the intracerebral semantic space from a language description acquired by performing natural language processing for the content of the new material on the basis of the intracerebral semantic space information stored by the semantic space storing unit; **characterized in that** the apparatus further comprises:
an object estimating unit (122) for estimating a second position corresponding to an object word in the intracerebral semantic space from the object word representing an object concept of the new material on the basis of the intracerebral semantic space information stored by the semantic space storing unit; and
an evaluation processing unit (123) for evaluating the new material on the basis of the first position estimated by the material estimating unit and the second position estimated by the object estimating unit.

## Patentansprüche

1. Materialbeurteilungsverfahren umfassend:
einen Hirnaktivitätsmessschritt (S101) zum Präsentieren eines Trainingsmaterials vor einem Testsubjekt und zum Messen der Hirnaktivität unter Verwendung einer Hirnaktivitätsmesseinheit;
einen semantischen Raumbildungsschritt (S103) zum Bilden eines intrazerebralen semantischen Raums, der eine intrazerebrale semantische Beziehung zwischen der Hirnaktivität und einem in einer Sprachbeschreibung erscheinenden Wort darstellt, auf der Grundlage eines Messergebnisses, das im Hirnaktivitätsmessschritt erworben wurde, und der Sprachbeschreibung, die durch Durchführen einer Verarbeitung natürlicher Sprache für den Inhalt des Trainingsmaterials unter Verwendung einer semantischen Raumbildungseinheit erworben wurde;
einen ersten Schätzschritt (S105) zum Schätzen einer ersten Position, die dem Inhalt eines neuen Materials im intrazerebralen semantischen Raum entspricht, aus einer Sprachbeschreibung, die durch Durchführen einer Verarbeitung natürlicher Sprache für den Inhalt des neuen Materials unter Verwendung einer Materialschätzeinheit erworben wurde; **dadurch gekennzeichnet, dass** das Verfahren ferner umfasst:
einen zweiten Schätzschritt (S107) zum Schätzen einer zweiten Position, die einem Objektwort im intrazerebralen semantischen Raum entspricht, aus dem Objektwort, das ein Objektkonzept des neuen Materials darstellt, unter Verwendung einer Objektschätzeinheit; und
einen Beurteilungsschritt (S108) zum Beurteilen des neuen Materials auf der Grundlage der im ersten Schätzschritt geschätzten ersten Position und der im zweiten Schätzschritt geschätzten zweiten Position unter Verwendung einer Beurteilungsverarbeitungseinheit.

2. Materialbeurteilungsverfahren nach Anspruch 1,
wobei im Beurteilungsschritt die Beurteilungsverarbeitungseinheit auf der Grundlage eines Abstands zwischen der ersten Position und der zweiten Position beurteilt, ob das neue Material dem Objektkonzept nahe kommt oder nicht.

3. Materialbeurteilungsverfahren nach Anspruch 1,
wobei im Beurteilungsschritt die Beurteilungsverarbeitungseinheit unter Verwendung eines inneren Produktwerts eines Vektors, der die erste Position repräsentiert, und eines Vektors, der die zweite Position repräsentiert, als einen Index beurteilt, ob das neue Material dem Objektkonzept nahe kommt oder nicht.

4. Materialbeurteilungsverfahren nach einem der Ansprüche 1 bis 3,
wobei im ersten Schätzschritt die Materialschätzeinheit eine Position im intrazerebralen semantischen Raum schätzt, die jedem der Wörter entspricht, die in der Sprachbeschreibung als erste Position umfasst sind.

5. Materialbeurteilungsverfahren nach einem der Ansprüche 1 bis 3,
wobei im ersten Schätzschritt die Materialschätzeinheit eine Position im intrazerebralen semantischen Raum für jedes der in der Sprachbeschreibung umfassten Wörter schätzt und den Schwerpunkt der Positionen der Wörter als die erste Position schätzt.

6. Materialbeurteilungsvorrichtung umfassend:
eine semantische Raumspeichereinheit (111), die intrazerebrale semantische Rauminformationen speichert, die einen intrazerebralen semantischen Raum darstellen, der auf der Grundlage eines Messergebnisses der Hirnaktivität, das durch Präsentieren eines Trainingsmaterials vor einem Testsubjekt unter Verwendung einer Hirnaktivitätsmesseinheit erworben wurde, und einer Sprachbeschreibung aufgebaut ist, die durch Durchführen einer Verarbeitung natürlicher Sprache für den Inhalt des Trainingsmaterials erworben wurde und eine intrazerebrale semantische Beziehung zwischen der Hirnaktivität und den in der Sprachbeschreibung erscheinenden Wörtern darstellt;
eine Materialschätzeinheit (121) zum Schätzen einer ersten Position, die dem Inhalt eines neuen Materials im intrazerebralen semantischen Raum entspricht, aus einer Sprachbeschreibung, die durch Ausführen einer Verarbeitung natürlicher Sprache für den Inhalt des neuen Materials erworben wurde, auf der Grundlage der durch die Speichereinheit für den semantischen Raum gespeicherten Informationen über den intrazerebralen semantischen Raum; **dadurch gekennzeichnet, dass** die Vorrichtung ferner umfasst:
eine Objektschätzeinheit (122) zum Schätzen einer zweiten Position, die einem Objektwort im intrazerebralen semantischen Raum entspricht, aus dem Objektwort, das ein Objektkonzept des neuen Materials darstellt, auf der Grundlage der durch die semantische Raumspeichereinheit gespeicherten intrazerebralen semantischen Rauminformationen; und
eine Beurteilungsverarbeitungseinheit (123) zum Beurteilen des neuen Materials auf der Grundlage der durch die Materialschätzeinheit geschätzten ersten Position und der durch die Objektschätzeinheit geschätzten zweiten Position.

## Revendications

1. Procédé d'évaluation de matériau comprenant :
une étape de mesure d'activité cérébrale (S101) consistant à présenter un matériau d'apprentissage à un sujet d'essai et mesurer l'activité cérébrale au moyen d'une unité de mesure d'activité cérébrale ;
une étape de construction d'espace sémantique (S103) consistant à construire un espace sémantique intracérébral représentant une relation sémantique intracérébrale entre l'activité cérébrale et un mot apparaissant dans une description de langage sur la base d'un résultat de mesure acquis dans l'étape de mesure d'activité cérébrale et la description du langage acquise par conduite d'un traitement de langage naturel pour le contenu du matériau d'apprentissage au moyen d'une unité de construction d'espace sémantique ;
une première étape d'estimation (S105) consistant à estimer une première position correspondant au contenu d'un nouveau matériau dans l'espace sémantique intracérébral à partir d'une description du langage acquise par conduite d'un traitement de langage naturel pour le contenu du nouveau matériau au moyen d'une unité d'estimation de matériau ; **caractérisé en ce que** le procédé comprend en outre :
une deuxième étape d'estimation (S107) consistant à estimer une deuxième position correspondant à un mot d'objet dans l'espace sémantique intracérébral à partir du mot d'objet représentant un concept d'objet du nouveau matériau au moyen d'une unité d'estimation d'objet ; et
une étape d'évaluation (S108) consistant à évaluer le nouveau matériau sur la base de la première position estimée dans la première étape d'estimation et de la deuxième position estimée dans la deuxième étape d'estimation au moyen d'une unité de traitement d'évaluation.

2. Procédé d'évaluation de matériau selon la revendication 1,
dans lequel, dans l'étape d'évaluation, l'unité de traitement d'évaluation évalue si le nouveau matériau est ou non proche du concept d'objet sur la base d'une distance entre la première position et la deuxième position.

3. Procédé d'évaluation de matériau selon la revendication 1,
dans lequel, dans l'étape d'évaluation, l'unité de traitement d'évaluation évalue si le nouveau matériau est ou non proche du concept d'objet au moyen d'une valeur de produit interne d'un vecteur représentant la première position et d'un vecteur représentant la deuxième position en tant qu'index.

4. Procédé d'évaluation de matériau selon l'une quelconque des revendications 1 à 3,
dans lequel, dans la première étape d'estimation, l'unité d'estimation de matériau estime une position dans l'espace sémantique intracérébral qui correspond à chacun des mots inclus dans la description de langage en tant que première position.

5. Procédé d'évaluation de matériau selon l'une quelconque des revendications 1 à 3,
dans lequel, dans la première étape d'estimation, l'unité d'estimation de matériau estime une position dans l'espace sémantique intracérébral pour chacun des mots inclus dans la description de langage et estime le centre de gravité des positions des mots comme étant la première position.

6. Appareil d'évaluation de matériau comprenant :
une unité de stockage d'espace sémantique (111) qui stocke des informations d'espace sémantique intracérébral représentant un espace sémantique intracérébral construit sur la base d'un résultat de mesure d'activité cérébrale acquis par présentation d'un matériau d'apprentissage à un sujet d'essai au moyen d'une unité de mesure d'activité cérébrale et d'une description du langage acquise par conduite d'un traitement de langage naturel pour le contenu du matériau d'apprentissage et représentant une relation sémantique intracérébrale entre l'activité cérébrale et les mots apparaissant dans la description de langage ;
une unité d'estimation de matériau (121), pour estimer une première position correspondant au contenu d'un nouveau matériau dans l'espace sémantique intracérébral à partir d'une description du langage acquise par conduite d'un traitement de langage naturel pour le contenu du nouveau matériau sur la base d'informations d'espace sémantique intracérébral stockées par l'unité de stockage d'espace sémantique, **caractérisé en ce que** l'appareil comprend en outre :
une unité d'estimation d'objet (122), pour estimer une deuxième position correspondant à un mot d'objet dans l'espace sémantique intracérébral à partir du mot d'objet représentant un concept d'objet du nouveau matériau sur la base des informations d'espace sémantique intracérébral stockées par l'unité de stockage d'espace sémantique ; et
une unité de traitement d'évaluation (123), pour évaluer le nouveau matériau sur la base de la première position estimée par l'unité d'estimation de matériau et de la deuxième position estimée par l'unité d'estimation d'objet.
